(19) Europäisches Patentamt
European Patent Office
Office européen des brevets

(11) EP 1 131 063 B1

(12) EUROPÄISCHE PATENTSCHRIFT

(45) Veröffentlichungstag und Bekanntmachung des Hinweises auf die Patenterteilung:
**23.04.2003 Patentblatt 2003/17**

(21) Anmeldenummer: **99958118.4**

(22) Anmeldetag: **24.11.1999**

(51) Int Cl.7: **A61K 31/122**, A61K 35/78, A61P 17/00, A61P 35/00

(86) Internationale Anmeldenummer:
**PCT/EP99/09067**

(87) Internationale Veröffentlichungsnummer:
**WO 00/030660 (02.06.2000 Gazette 2000/22)**

(54) **HYPERFORIN-SALBE ODER -CREME**

HYPERFORIN OINTMENT OR CREAM

POMMADE OU CREME A BASE D'HYPERFORINE

(84) Benannte Vertragsstaaten:
**AT BE CH CY DE DK ES FI FR GB GR IE IT LI LU MC NL PT SE**

(30) Priorität: **25.11.1998 DE 19854446**
**24.03.1999 DE 19913333**

(43) Veröffentlichungstag der Anmeldung:
**12.09.2001 Patentblatt 2001/37**

(60) Teilanmeldung:
**02022953.0 / 1 275 382**

(73) Patentinhaber: **Universitätsklinikum Freiburg**
**79106 Freiburg (DE)**

(72) Erfinder:
- **SIMON, Jan, C.**
  **D-79249 Merzhausen (DE)**
- **SCHEMPP, Christoph, M.**
  **D-79102 Freiburg (DE)**
- **SCHÖPF, Erwin**
  **D-79104 Freiburg (DE)**
- **SIMON-HAARHAUS, Birgit**
  **D-79194 Gundelfingen (DE)**

(74) Vertreter: **Keller, Günter, Dr. et al**
**Lederer & Keller**
**Patentanwälte**
**Prinzregentenstrasse 16**
**80538 München (DE)**

(56) Entgegenhaltungen:
**EP-A- 0 424 534**
**EP-A- 0 599 307**
**WO-A-91/15218**
**WO-A-95/11035**
**WO-A-97/39355**
**CH-A- 680 570**
**DE-A- 19 700 788**
**GB-A- 2 311 009**
**RU-C- 2 013 093**
**RU-C- 2 044 547**
**US-A- 4 911 925**

- **DATABASE BIOSIS [Online] BIOSCIENCES INFORMATION SERVICE, PHILADELPHIA, PA, US; Mikrobiologicheskii Zhurnal (Kiev), 1978 AIZENMAN B E: " HIGHER PLANTS AS A SOURCE FOR PRODUCTION OF NEW ANTIBIOTICS" XP002139268 & AIZENMAN B E: "HIGHER PLANTS AS A SOURCE FOR PRODUCTION OF NEW ANTIBIOTICS" MIKROBIOLOGICHESKII ZHURNAL (KIEV), Bd. 40, Nr. 2, 1978, Seiten 233-241, XP000913495**
- **DATABASE BIOSIS [Online] BIOSCIENCES INFORMATION SERVICE, PHILADELPHIA, PA, US; Lietuvos TSR Mokslu Akademijos Darbai Serija C, 1986 VALAVICHYUS YU M; IVANAUSKAS V P; YASKONIS YU A: "ANTITUMOR ACTIVITY OF MEDICINAL PLANTS FROM THE LITHUANIAN SSR USSR 6. COMMON ST.-JOHN'S-WORT AND CHAMOMILLA-RECUTITA" XP002139269 & VALAVICHYUS YU M; IVANAUSKAS V P; YASKONIS YU A: "ANTITUMOR ACTIVITY OF MEDICINAL PLANTS FROM THE LITHUANIAN SSR USSR 6. COMMON ST.-JOHN'S-WORT AND CHAMOMILLA-RECUTITA" LIETUVOS TSR MOKSLU AKADEMIJOS DARBAI SERIJA C BIOLOGIJOS MOKSLAI, Nr. 3, 1986, Seiten 110-113, XP000913615**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- CHAVEZ M.L., CHAVEZ P.L.: "Monographs on alternative therapies: Saint John's Wort" HOSPITAL PHARMACY, Bd. 32, Nr. 12, 1997, Seiten 1621-1632, XP000913486
- DECOSTERD L A; STOECKLI-EVANS H; CHAPUIS J-C; MSONTHI J D; SORDAT B; HOSTETTMANN K: "NEW HYPERFORIN DERIVATIVES FROM HYPERICUM-REVOLUTUM VAHL WITH GROWTH-INHIBITORY ACTIVITY AGAINST A HUMAN COLON CARCINOMA CELL LINE" HELVETICA CHIMICA ACTA, Bd. 72, Nr. 3, 1989, Seiten 464-471, XP000907328
- STEPANOVA E; SARAF A; SUKOLIN G : "Treatment of atopic dermatitis with original natural creams. " ALLERGY (COPENHAGEN) , Bd. 51, Nr. SUPPL. 31, 1996, Seite 167 XP000933496
- REYNOLDS J.E.F.: "Martindale, The Extra Pharmacopoeia, Thirtieth Edition" 1993 , PHARMACEUTICAL PRESS , LONDON, GB XP002143407 212040 Seite 1378, Spalte 3 Seite 1902, Spalte 2, Absatz 12
- DATABASE WPI Week 199731 Derwent Publications Ltd., London, GB; AN 1997-340135 XP002143409 PISHKINA K I : "Herbal composition for treating mastitis in lactating animals - including extracts of calendula, nettle, plantain,Origanum, St. John's wort, thyme, linden and celandine, emulsifier and vegetable oil " & RU 2 070 409 A (PISHKINA K I), 20. Dezember 1996 (1996-12-20)
- CHEMICAL ABSTRACTS, vol. 131, no. 25, 20. Dezember 1999 (1999-12-20) Columbus, Ohio, US; abstract no. 341773, INOMATA, SHINJI ET AL: "Antiaging agents containing extracts of Hypericaceae plant and antiaging cosmetics" XP002143408 & PATENT ABSTRACTS OF JAPAN vol. 2000, no. 02 & JP 11 315008 A (SHISEIDO CO LTD), 16. November 1999 (1999-11-16) & JP 11 315008 A (SHISEIDO CO., LTD., JAPAN) 16. November 1999 (1999-11-16)

## Beschreibung

Die vorliegende Erfindung betrifft eine Hyperforin-Salbe oder -Creme, die sich als Applikationsform für das Hyperforin eignet.

Hyperforin zählt neben den, Hyperizinen zu den charakteristischen Inhaltsstoffen des Johanniskrauts (Hypericum perforatum L.), das darüber hinaus allgemein im Pflanzenreich vorkommende Inhaltsstoffe wie Flavon- und Flavonolderivate, Rutin, Hyperosid, Xanthonderivate, Amentoflavon, Biapigenin und ätherische Öle enthält.

Johanniskraut und Johanniskrautauszüge werden schon seit geraumer Zeit in der Medizin und der Volksmedizin als Arzneimittel für verschiedenste Indikationen eingesetzt. Neuerdings wird der Bestandteil Hypericin als Wirkstoff auch isoliert in Arzneimitteln verwendet (L. Roth, Hypericum, Hypericin, Ecomed Arzneipflanzen-Monographie, Ecomed, Landsberg/Lech, 1990).

Die Monographie "Hyperici herba (Johanniskraut)", die von der Kommission E des früheren Gesundheitsamtes am 5.12.1984 publiziert wurde, nennt als Anwendungsgebiet für Hypericum-Präparate (intern als Tropfen oder Tabletten) : "Psychovegetative Störungen, depressive Verstimmungszustände, Angst und/oder nervöse Unruhe". In zahlreichen plazebokontrollierten Studien wurde die den trizyklischen Antidepressiva vergleichbare antidepressive Wirksamkeit des Johanniskrauts belegt.

Als Hausmittel wird insbesondere das Johanniskrautöl zur Wundund Schmerzbehandlung und bei Verbrennungen eingesetzt (L. Roth a.a.O.).

Als Wirkstoff des Johanniskrautöls wurde zunächst aufgrund der charakteristischen roten Farbe und Fluoreszenz des Öls Hypericin angenommen. Die Formel von Hypericin ist im folgenden wiedergegeben:

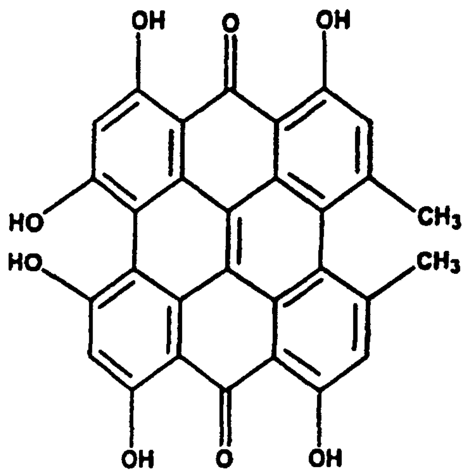

Neuere Untersuchungen der Zusammensetzung von Johanniskrautöl haben jedoch ergeben, daß es tatsächlich kein Hypericin enthält, sondern sog. Ölhypericine, welche lipophile Lyseprodukte des Hypericins darstellen. Außerdem enthält Johanniskrautöl Hyperforin (P. Maisenbacher et al., Planta Med. 58:351-354 (1992) und B. Hellwig, DAZ 137, 29, Seiten 35-36), dessen Formel im folgenden wiedergegeben ist:

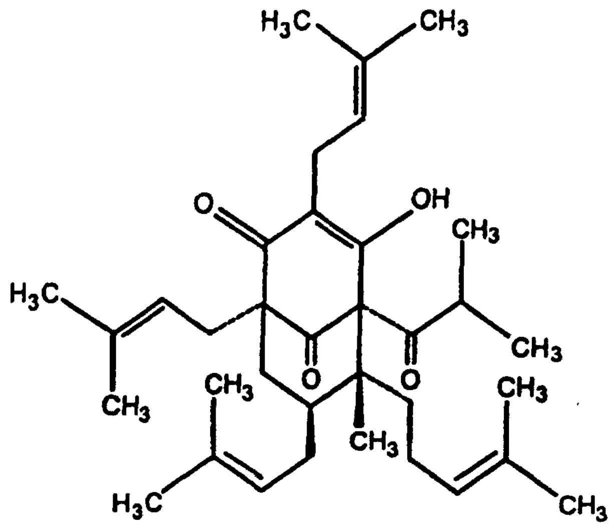

Hyperforin hat als Wirkstoff Interesse als antidepressive Substanz erweckt (Pharmacopsychiatry 1998, Vol. 31, Supplement 1, Seiten 1-60). Darüberhinaus ist Hyperforin antibakteriell wirksam (A. I. Gurevich et al., L. Antibiotiki, 16:510-513 (1971)).

Stabile Extrakte aus Hypericum perforatum L. sind aus der DE 197 14 450 und der DE 196 46 977 bekannt.

Die DE 195 47 317 offenbart ein antivirales Medikament auf der Basis von Wirkstoffen des Johanniskrauts, das 1-50% Hypericin oder Pseudohypericin enthält.

Die EP-A-0 599 307 offenbart einen Trockenextrakt aus Johanniskraut mit erhöhtem Hyperforin-Gehalt sowie dessen Verwendung zur Herstellung psychovegetativ und antidepressiv wirksamer Arzneimittel.

Neben Johanniskrautöl sind Salben bekannt, die neben verschiedenen anderen Heilkräutern Hypericum in geringer Konzentration enthalten. Hierzu gehören beispielsweise "Unguentum Truw" zur Wundbehandlung und die Homöopatika "Traumeel S", ein Antiphlogistikum, und "Atemaron N R30", ein Analgetikum/Antirheumatikum. Es ist nicht bekannt, ob diese Salben Hyperforin enthalten. Wegen der Instabilität von Hyperforin muß jedoch davon ausgegangen werden, daß Hyperforin nicht oder nur in geringen Mengen enthalten ist, wenn der Extrakt nicht unter Lichtschutz und Oxidationsschutz hergestellt, aufbewahrt und verarbeitet ist. In einem handelsüblichen Hypericum-Extrakt (Hyperforat) z.B. ist Hyperforin nicht nachweisbar.

Bei der topischen Anwendung und insbesondere bei der Behandlung entzündlicher Hauterkrankungen hat das bekannte Johanniskrautöl den Nachteil, daß es aufgrund seines Fettgehalts nur in begrenztem Umfang einsetzbar ist und sich beispielsweise für die Behandlung von Ekzemen nicht eignet. Darüber hinaus sind nur fettlösliche Bestandteile des Johanniskrauts in dem Öl enthalten, nicht jedoch beispielsweise Hypericin.

Bei der Aufbereitung des Wirkstoffs muß beachtet werden, daß es sich um eine lichtempfindliche Substanz handelt, die sich leicht zersetzt. Daher muß bei der Gewinnung, Lagerung und Verabreichung auf einen entsprechenden Lichtschutz geachtet werden.

Es wurde gefunden, daß sich Hyperforin vorteilhaft in einer pharmazeutischen Formulierung in Form einer topischen Salbe oder Creme, die mindestens 15 µg/ml Hyperforin enthält, verabreichen läßt.

Die Salbe oder Creme sollte eine möglichst hohe Wirkstoffkonzentration enthalten, bevorzugt im Bereich von 0,02-20 mg/ml Hyperforin, bevorzugter im Bereich von 1-20 mg/ml und besonders bevorzugt etwa 10 mg/ml (1% Hyperforin).

Neben dem Wirkstoff Hyperforin kann die erfindungsgemäße Salbe oder Creme zusätzlich Hypericine als weitere Wirkstoffe enthalten. Die Konzentration der Hypericine in der Salbe oder Creme sollte dabei in Summe mindestens 15 µg/ml, bevorzugt 20-150 µg/ml betragen. Unter Hypericinen werden vorliegend Hypericin und dessen pharmazeutisch wirksamen Derivate verstanden. Hierzu gehört insbesondere Pseudohypericin, das sich von Hypericin dadurch unterscheidet, daß eine Methylgruppe durch Hydroxymethyl ersetzt ist.

Die genannten Wirkstoffe können entweder als Reinsubstanzen in die Salbe oder Creme eingebracht werden oder in Form eines Johanniskrautextrakts definierter Konzentration. Die erfindungsgemäße Salbe oder Creme umfaßt dabei bevorzugt außer Johanniskrautextrakt keine weiteren Pflanzenextrakte.

Beispielsweise eignet sich zur Herstellung der erfindungsgemäßen Salbe oder Creme ein Johanniskrautextrakt, der mindestens 200 µg/ml Hyperforin und mindestens 200 µg/ml Hypericine enthält. Bevorzugt enthält der verwendete Extrakt 200-100.000 µg/ml, insbesondere etwa 1000 µg/ml Hyperforin und 200-1000 µg/ml Hpericine. Bei diesen Wirkstoffkonzentrationen im Extrakt sollte die erfindungsgemäße Salbe oder Creme mindestens 5 Gew.-% des Extrakts enthalten. Vorteilhaft kann eine erfindungsgemäße Salbe beispielsweise etwa 15 Gew.-% des Extrakts enthalten und eine erfindungsgemäße Creme etwa 10 Gew.-% des Extrakts.

Der auf Hyperforin und Hypericin standardisierte Gesamtextrakt sollte ein ethanolischer oder ein mit Ethanol versetzter Extrakt sein. Hierbei kann es sich beispielsweise um im Handel erhältliche Gesamtextrakte (Tinkturen) handeln. Der Ethanolgehalt liegt bevorzugt zwischen 20 und 60 % v/v, bevorzugt bei 40-50% v/v. Diese Anforderungen erfüllt beispielsweise ein Gesamtextrakt der Firma Caelo, der erfindungsgemäß bevorzugt eingesetzt wird und der 240 µg/ml Hyperforin und 300 µg/ml Hypericin enthält.

Prinzipiell sind auch wäßrige Extrakte, $CO_2$-Extrakte oder Frischpflanzenauszüge geeignet, insofern sie die Anforderungen an den Wirkstoffgehalt erfüllen.

Die qualitative und quantitative Analyse des für die erfindungsgemäße Salbe oder Creme verwendeten Johanniskrautextrakts erfolgt mittels Hochdruck-Flüssigkeits-Chromatographie (HPLC) (P. Maisenbacher et al., Planta Med. 58:351-354 (1992)). Zur Messung der Gesamthypericine wird das photometrische Verfahren nach dem "Deutschen-Arzneimittel-Codex" (DAC) verwendet.

Die erfindungsgemäße Salbe oder Creme kann neben dem Wirkstoff oder den Wirkstoffen verschiedene pharmazeutisch verträgliche Creme- bzw. Salbengrundlagen umfassen. Hierzu gehören beispielsweise weiße Vaseline, dickflüssiges Paraffin, Wollwachs, Ascorbylpalmitat, Glycerolmonostearat 60, Tocopherol (Vitamin E), Cetylalkohol, mittelkettige Triglyceride, gelbes Wachs, Propylenglycol, Macrogol-1000-glycerolmonostearat, Zitronensäure, Ascorbinsäure und andere Konservierungsstoffe und destilliertes Wasser.

Eine bevorzugte erfindungsgemäße Salbe umfaßt etwa 15 Gew.-% Johanniskrautextrakt sowie weiße Vaseline, dickflüssiges Paraffin, Wollwachs und Ascorbylpalmitat jeweils in geeigner Menge.

Eine bevorzugte erfindungsgemäße Creme umfaßt etwa 10 Gew.-% Johanniskrautextrakt sowie weiße Vaseline, Glycerolmonostearat 60, Cetylalkohol, mittelkettige Triglyceride, gelbes Wachs, Propylenglykol, Macrogol-1000-glycerolmonostearat, Zitronensäure und Wasser jeweils in geeigneter Menge.

Die vorliegende Erfindung betrifft auch ein Verfahren zur Herstellung einer topischen Salbe oder Creme, worin man Hyperforin und gegebenenfalls Hypericine oder einen Johanniskrautextrakt, der mindestens 200 µg/ml Hyperforin und mindestens 200 µg/ml Hypericine enthält, so mit üblichen pharmazeutisch verträglichen Hilfsstoffen vermischt, daß man eine Salbe oder Creme mit einem Mindestgehalt von 15 µg/ml Hyperforin und gegebenenfalls mindestens 15 µg/ml Hypericine erhält.

Bei Verwendung eines Johanniskrautextrakts zur Herstellung der erfindungsgemäßen Salbe oder Creme muß dieser zum Schutz des Hyperforins vor Oxidation bis zu seiner Verarbeitung unter Schutzgas (z.B. Argon) fest verschlossen und lichtgeschützt aufbewahrt werden.

Die erfindungsgemäße Salbe oder Creme eignet sich beispielsweise zur Behandlung von entzündlichen Hauterkrankungen, Altershaut und bakteriellen Hautinfektionen. Die Salbe ist aufgrund der fetten Grundlage insbesondere bei trockenen, schuppenden Hautveränderungen, die mit Juckreiz oder Entzündungen einhergehen, indiziert. Der bevorzugte Wirkstoffgehalt der Salbe (15 Gew.-%) ist etwas höher als der bevorzugte Wirkstoffgehalt der Creme (10 Gew.-%), die sich aufgrund des amphiphilen Charakters besonders zur Behandlung akuter bis subakuter ekzematöser Hautveränderungen eignet.

Die erfindungsgemäße Salbe eignet sich somit insbesondere zur Behandlung von chronischen, auch superinfizierten Ekzemen, Exsikkationsekzemen, hyperkeratotischen Hand- und Fußekzemen, subakute bis chronische atopische Dermatitis (Neurodermitis), Lichen simplex, Kontaktekzemen, Prurigo simplex subacuta und andere Prurigo-Arten und Psoriasis vulgaris vom Plaque-Typ sowie der Altershaut.

Die Creme eignet sich insbesondere zur Behandlung akuter bis subakuter atopischer Dermatitis (Neurodermitis), akuten bis subakuten Kontaktekzemen, Psoriasis guttata und der Altershaut sowie zur Nachbehandlung und Rezidivprophylaxe aller Ekzeme.

Die Salbe und die Creme können auch in der Veterinärmedizin beispielsweise zur Behandlung entzündlicher und infizierter Hautkrankheiten, wie Mastitis (Euterentzündung), verwendet werden.

Hyperforin wirkt ab Konzentrationen von 100 ng/ml proliferationshemmend auf humane Keratinozyten und Lymphozyten. Darüber hinaus konnte nachgewiesen werden, daß Hypericin proliferationshemmend auf Keratinozyten (HaCaT) und T-Zellen wirkt und in diesen Zellen Apoptose induzieren kann. Diese Wirkung wird teilweise durch die Bildung von Sauerstoffradikalen vermittelt.

Wegen der möglichen Photosensibilisierung aufgrund des optionalen Hypericin-Gehalts der erfindungsgemäßen Salbe oder Creme wurde untersucht, ob die lokale Anwendung des erfindungsgemäßen Hypericin-haltigen Präparats zu sonnenbrandähnlichen Erscheinungen führen kann. Diese Untersuchungen zeigten kein Sonnenbrandrisiko.

Die erfindungsgemäße Salbe oder Creme hat den Vorteil, daß sie von der Grundlage her an verschiedene Hautzustände angepaßt werden kann. So eignet sich die Creme besonders zur Behandlung akuter und subakuter

Dermatosen, die Salbe zur Behandlung chronischer Dermatosen. Darüber hinaus können sowohl fettlösliche (Hyperforin) als auch wasserlösliche (Hypericin) Wirkstoffe des Johanniskrauts in eine Salben- bzw. Cremegrundlage eingearbeitet werden. Hierdurch kann eine überlegene Wirkung gegenüber dem bekannten Johanniskrautöl erreicht werden. Außerdem ist die Penetration der Wirkstoffe aus Creme- und Salbengrundlagen im Vergleich zu ölen besser.

Durch die erfindungsgemäße Salbe oder Creme zur lokalen topischen Anwendung wird das Therapiespektrum entzündlicher Hautkrankheiten, wie z.B. Neurodermitis, entscheidend bereichert. Insbesondere besteht die Möglichkeit der Reduktion der Cortison-Therapie.

Figur 1 zeigt die proliferationshemmende Wirkung von Hyperforin auf HaCaT-Zellen in vitro (Beispiel 7).

Figur 2 zeigt die proliferationshemmende Wirkung von Hyperforin auf PBMC in vitro (Beispiel 8).

Figur 3 zeigt die proliferationshemmende Wirkung einer auf Hyperforin standardisierten Creme und einer Hyperforin-Creme im Vergleich zum immunsuppressiven Effekt einer Sonnensimulator-Bestrahlung (zweifache MED). Als Kontrolle wurde unbehandelte Haut getestet (Beispiel 9).

Figur 4 zeigt die proliferationshemmende Wirkung von Hyperforin in vitro (Beispiel 10).

Die nachfolgenden Beispiele sollen die Erfindung näher erläutern. Die Reinheit des Hyperforins betrug über 90%. In allen Versuchen wurde das Lösungsmittel DMSO in der maximalen verwendeten Konzentration getestet und zeigte keinerlei Effekte auf Proliferation oder Apoptoserate.

Beispiel 1

Eine Johanniskrautsalbe wurde nach folgender Rezeptur hergestellt (Angaben in Gew.-%):

| | |
|---|---|
| Weiße Vaseline | 50.0 |
| Dickflüssiges Paraffin | 9.0 |
| Wollwachs | 25.0 |
| Ascorbylpalmitat | 1.0 |
| Johanniskrautextrakt | 15.0 |
| | 100 |

Die Vaseline, das dickflüssige Paraffin und das Wollwachs wurden im Wasserbad auf 60°C erwärmt und gemischt. Man ließ unter Rühren abkühlen, wobei das Ascorbylpalmitat eingearbeitet wurde, und nach dem Abkühlen wurde das Johanniskrautextrakt (Gesamtextrakt der Firma Caelo (Hyperforin 240 µg/ml, Hypericin 300 µg/ml) eingearbeitet. Die so erhaltene Salbe enthielt 36 µg/ml Hyperforin und 45 µg/ml Hypericin.

Beispiel 2

Nach folgender Rezeptur wurde eine Johanniskrautcreme hergestellt (Angaben in Gew.-%) :

| | |
|---|---|
| Weiße Vaseline | 20.0 |
| Glycerolmonostearat 60 | 4.0 |
| Cetylalkohol | 6.0 |
| Mittelkettige Triglyceride | 8.0 |
| Gelbes Wachs | 4.0 |
| Propylenglykol | 10.0 |
| Macrogol-1000-glycerolmonostearat | 7.0 |
| Zitronensäure | 1.0 |
| Destilliertes Wasser | 30.0 |
| Johanniskrautextrakt | 10.0 |
| | 100 |

Die Vaseline, das Glycerolmonostearat 60, der Cetylalkohol, die mittelkettigen Triglyceride und das gelbe Wachs wurden im Wasserbad auf 60°C erhitzt und gemischt. Separat wurde das Macrogol-1000-glycerolmonostearat, das Propylenglykol, das Wasser und die Zitronensäure im Wasserbad auf 60°C erhitzt und dann in die erste Mischung eingearbeitet. Es wurde bis zum Erkalten gerührt und anschließend wurde der Johanniskrautextrakt (Gesamtextrakt der Firma Caelo (Hyperforin 240 µg/ml, Hypericin 300 µg/ml)) eingearbeitet. Die so erhaltene Creme enthielt 24 µg/ml Hyperforin und 30 µg/ml Hypericin.

Beispiel 3

Zur Herstellung einer Hyperforin-Salbe bzw. -Creme mit höherer Wirkstoffkonzentration wurde ein mit Neutralöl versetzter Extrakt der Firma Flavix (Rehlingen) mit einem Hyperforingehalt von 20 Gew.-% (20 g/100 g) verwendet. Hierzu wurden 5 g des Extrakts in 10 ml 70%-igem Ethanol aufgelöst, um eine Ausgangslösung mit einer Hyperforinkonzentration von 100 mg/ml zu erhalten. Diese Ausgangslösung wurde an Stelle des Johanniskrautextrakts der Firma Caelo in der in Beispiel 1 bzw. 2 beschriebenen Weise in eine Salben- bzw. Cremegrundlage eingearbeitet. Es wurden Salben und Cremen folgender Rezepturen hergestellt (Angaben in Gew.-%):

a) Salbe

| | **1% Hyperforin** | **0,1% Hyperforin** |
|---|---|---|
| Weiße Vaseline | 50,0 | 50,0 |
| Dickflüssiges Paraffin | 9,0 | 9,0 |
| Wollwachs | 30,0 | 39,0 |
| Ascorbylpalmitat | 1,0 | 1,0 |
| Ausgangslösung | 10,0 | 1,0 |
| | 100,0 | 100,0 |

b) Creme

| | 1% Hyperforin | 0,1% Hyperforin |
|---|---|---|
| Weiße Vaseline | 20,0 | 20,0 |
| Glycerolmonostearat | 4,0 | 4,0 |
| Cetylalkohol | 6,0 | 6,0 |
| Mittelkettige Triglyceride | 8,0 | 17,0 |
| Gelbes Wachs | 4,0 | 4,0 |
| Propylenglykol | 10,0 | 10,0 |
| Macrogol-1000-glycerolmonostearat | 7,0 | 7,0 |
| Zitronensäure | 1,0 | 1,0 |
| Destilliertes Wasser | 30,0 | 30,0 |
| Ausgangslösung | 10,0 | 1,0 |
| | 100,0 | 100,0 |

Beispiel 4

Die immunomodulatorischen Wirkungen der erfindungsgemäßen Präparation auf die Haut wurden ex vivo am Menschen (3x4 Probanden) nach Anwendung der Johanniskrautsalbe aus Beispiel 1 im Vergleich zu Johanniskrautöl untersucht. Hierzu wurden bei freiwilligen Probanden unterschiedlich behandelte Hautproben entnommen und es wurde untersucht, ob die Fähigkeit epidermaler Langerhans-Zellen, Antigen zu präsentieren, beeinflußt wird.

Im einzelnen wurde folgende MECLR (mixed epidermal cell leukocyte reaction) durchgeführt: Bei jeweils 4 Probanden wurden an den Unterarm-Beugeseiten runde Testfelder mit 2 cm Durchmesser mit Johanniskrautöl, Johanniskrautcreme oder der Behandlungsgrundlage behandelt und der Effekt im Vergleich zu unbehandelter Haut untersucht. 100 µl der Testsubstanzen wurden für 24h in Epikutantestkammern appliziert. Danach wurden die Rückstände entfernt und mittels eines Vakuums epidermale Saugblasen erzeugt. Das Blasendach wurde steril mit dem Skalpell abpräpariert und es wurde durch Trypsinbehandlung eine Epidermalzellsuspension (EC) hergestellt. 50.000 EC wurden mit 150.000 T-Zellen (TC) in RPMI-1640 mit 10% fötalem Kälberserum (FCS) mit 1% Penicillin/Streptomycin (alles Gibco) in 96-well Flachboden Mikrotiterplatten (Greiner) für 6 Tage kokultiviert (37°C, 5% $CO_2$). Dann wurde $3{,}7 \cdot 10^4$

Bq (1 µCurie) well $^3$H-Thymidin (Amersham) zugegeben und die inkorporierte Radioaktivität in einem Szintillationscounter (Canberra-Packard) gemessen. Die gemessene Radioaktivität ist proportional zur DNA-Replikation der Zellen.

Diese Untersuchungen zeigten, daß es durch die erfindungsgemäße Johanniskrautsalbe zu einer Proliferationshemmung kommt. Bei der Anwendung von Johanniskrautöl hingegen kommt es zu einer Proliferationssteigerung.

Diese Ergebnisse sprechen für eine antientzündliche Wirkung der erfindungsgemäßen Johanniskrautsalbe, die beim Öl nicht nachweisbar war.

Beispiel 5

Bei einem Patienten mit Ekzem wurde in einem Halbseitenversuch ein Unterschenkel für zwei Wochen mit Johanniskrautöl, der andere mit der erfindungsgemäßen Johanniskrautsalbe gemäß Beispiel 1 behandelt. Der mit der Salbe behandelte Unterschenkel war bei der Kontrolle sehr gut gebessert, der mit dem Öl behandelte Unterschenkel eher verschlechtert.

Beispiel 6

Es wurden 4 Patienten mit verschiedenen lokalisierten Ekzemformen behandelt. Nach Aufklärung und Einwilligung erfolgte eine Fotodokumention und die Patienten führten über 2 Wochen eine Monotherapie mit der erfindungsgemäßen Salbe durch. Danach wurde eine erneute Fotokokumentation durchgeführt. Bei zwei Patienten kam es zu einer vollständigen Abheilung, bei den anderen Patienten zu einer deutlichen Besserung des Befundes. Die Ergebnisse dieser Behandlung sind in Tabelle 1 zusammengefaßt.

Tabelle 1

| Nr. | Geschlecht | Diagnose | Behandlung | Therapieerfolg |
|---|---|---|---|---|
| 1 | weiblich | Beugenekzem | Hypericum-Creme | abgeheilt |
| 2 | männlich | Handekzem | Hypericum-Creme | gebessert |
| 3 | weiblich | Neurodermitis Arme | Hypericum-Creme | abgeheilt |
| 4 | weiblich | Prurigo Unterschenkel | Hypericum-Creme | gebessert |

Beispiel 7

Dieses Beispiel zeigt die proliferationshemmende Wirkung von Hyperforin auf Keratinozyten. HaCaT-Zellen wurden in Keratinozyten-Medium mit 10% fötalem Kälberserum (FCS) mit 1 % Penicillin/Streptomycin (alles Gibco) kultiviert (37° C, 5% $CO_2$). Subkonfluente Kulturen wurden mit EDTA-Trypsin (Gibco) abgelöst, 3x in PBS gewaschen und in einer Dichte von 20.000/well in 96-well Flachboden Mikrotiterplatten (Greiner) weiter für 24h kultiviert (bis zur Adhärenz). Danach wurde frisch in DMSO gelöstes Hyperforin (HWI-Analytik) für 24h dazugegeben. Dann wurde $3{,}7 \cdot 10^4$ Bq (1 µCurie) well $^3$H-Thymidin (Amersham) zugegeben und die inkorporierte Radioaktivität in einem Szintillationscounter (Canberra-Packard) gemessen. Die gemessene Radioaktivität ist proportional zur DNA-Replikation der Zellen. Die Ergebnisse für Hyperforinkonzentrationen von 0 bis 100 µg/ml sind in Figur 1 wiedergegeben, wobei cpm counts per minute bedeutet. Es zeigt sich, daß es bei einer Hyperforinkonzentration von 100 µg/ml zu einer praktisch vollständigen Proliferationshemmung kommt.

Beispiel 8

Dieses Beispiel zeigt die proliferationshemmende Wirkung von Hyperforin auf mononukleäre Zellen des peripheren Blutes (PBMC). PBMC wurden durch Dichtegradient-Zentrifugation mit Ficoll (Seromed) aus heparinisiertem Blut gewonnen. Die PBMC wurden 3x in PBS gewaschen und in RPMI-1640 mit 10% fötalem Kälberserum (FCS) mit 1% Penicillin/Streptomycin (alles Gibco) in einer Dichte von 200.000/well in 96-well Flachboden Mikrotiterplatten (Greiner) für 24h kultiviert (37° C, 5% $CO_2$). Danach wurden die Zellen mit 1 µg/ml Phytohämagglutinin (PHA) (Wellcome) stimuliert und es wurde frisch in DMSO gelöstes Hyperforin (HWI-Analytik) für 24h dazugegeben. Dann wurde $3{,}7 \cdot 10^4$ Bq (1 µCurie) well $^3$H-Thymidin (Amersham) zugegeben und die inkorporierte Radioaktivität in einem Szintillationscounter (Canberra-Packard) gemessen. Die gemessene Radioaktivität ist proportional zur DNA-Replikation der Zellen. Die Ergebnisse für Hyperforinkonzentrationen von 0 bis 100 µg/ml sind in Figur 2 wiedergegeben, wobei cpm counts per minute bedeutet. Es zeigt sich, daß bereits geringe Konzentrationen von Hyperforin proliferationshemmend auf PBMC wirken und es bei einer Konzentration von 100 µg/ml zu einer praktisch vollständigen Proliferationshemmung

kommt.

Beispiel 9

Dieses Beispiel zeigt eine immunmodulatorische Wirkung von Hyperforin-Creme mit hohem Hyperforinanteil in vivo. Es konnte durch Experimente mit aufgereinigtem Hyperforin gezeigt werden, daß diese immunmodulatorische Wirkung auf Hyperforin zurückzuführen ist.

Die immunmodulatorische Wirkung der erfindungsgemäßen Creme wurde ex vivo am Menschen (je 4 Probanden) getestet. Hierzu wurden unterschiedlich behandelte Hautproben entnommen und es wurde untersucht, ob die Fähigkeit epidermaler Langerhans-Zellen, Antigen zu präsentieren beeinflußt wird. Im einzelnen wurde dies in einer MECLR (mixed epidermal cell leukocyte reaction) untersucht: Bei je 4 Probanden wurden an der Unterarm-Beugeseite runde Testfelder mit 2 cm Durchmesser mit Hypericum-Creme (mit 24 µg/ml Hyperforin und 30 µg/ml Hypericin), mit Hyperforin-Creme (24 µg/ml) oder mit Sonnensimulator-Bestrahlung (144 J/cm$^2$) behandelt. Als Kontrollen dienten unbehandelte Haut und Anwendung des Vehikels ohne Wirkstoffe. 100 µl der Testsubstanzen wurden für 24h in Epikutantestkammern appliziert. Danach wurden die Rückstände entfernt und mittels eines Vakuums eine epidermale Saugblase erzeugt. Das Blasendach wurde steril mit dem Skalpell abpräpariert und es wurde durch Trypsinbehandlung eine Epidermalzellsuspension (EC) hergestellt. 50.000 EC wurden mit 150.000 TC (T-Zellen) in 1640-RPMI mit 10% fötalem Kälberserum (FCS) mit 1% Penicillin-Streptomycin in 96-well Flachboden Mikrotiterplatten (Greiner) für 6 Tage kokultiviert (37°C, 5% $CO_2$). Dann wurde $3{,}7 \cdot 10^4$ Bq (1 µCurie) well $^3$H-Thymidin (Amersham) zugegeben und nach 18h die inkorporierte Radioaktivität in einem Szintillationscounter (Canberra-Packard) gemessen. Die gemessene Radioaktivität ist proportional zur DNA-Replikation der Zellen.

Die Ergebnisse (Figur 3) zeigen, daß die Hyperforin-haltige Johanniskrautcreme und die Hyperforin-Creme eine signifikante Hemmung der Proliferation in der Größenordnung der Bestrahlung mit dem Sonnensimulator bewirken.

Beispiel 10

Dieses Beispiel zeigt die proliferationshemmende Wirkung von Hyperforin in vitro. In allen Fällen wurde reines Hyperforin der Firma HWI-Analytik (Rheinzabern) verwendet. Die Reinheit des Hyperforins betrug > 90%. In allen in vitro-Versuchen wurde das Lösungsmittel DMSO in der maximalen verwendeten Konzentration getestet und zeigte keinen Effekt auf Proliferation und Vitalität der Zellen. Es wurden normale Hautproben gesunder Probanden entnommen, diese wurden in vitro mit Hyperforin inkubiert und es wurde untersucht, ob die Fähigkeit epidermaler Langerhans-Zellen, Antigen zu präsentieren beeinflußt wird.

Dies wurde wie in Beispiel 8 in einer MECLR (mixed epidermal cell leukocyte reaction) untersucht: Bei je 4 Probanden wurden an der Unterarm-Beugeseite mittels eines Vakuums epidermale Saugblasen erzeugt. Das Blasendach wurde steril mit dem Skalpell abpräpariert und es wurde durch Trypsinbehandlung eine Epidermalzellsuspension (EC) hergestellt. Es wurde jeweils ein Teil der EC oder TC für 24h mit 24 µg/ml Hyperforin inkubiert. Danach wurden die Zellen gewaschen, und es wurden 50.000 EC mit 150.000 TC (T-Zellen) in 1640-RPMI mit 10% fötalem Kälberserum (FCS) mit 1% Penicillin-Streptomycin in 96-well Flachboden Mikrotiterplatten (Greiner) für 6 Tage kokultiviert (37°C, 5% $CO_2$). Dann wurde $3{,}7 \cdot 10^4$ Bq (1 µCurie) well $^3$H-Thymidin (Amersham) zugegeben und nach 18h die inkorporierte Radioaktivität in einem Szintillationscounter (Canberra-Packard) gemessen. Die gemessene Radioaktivität ist proportional zur DNA-Replikation der Zellen.

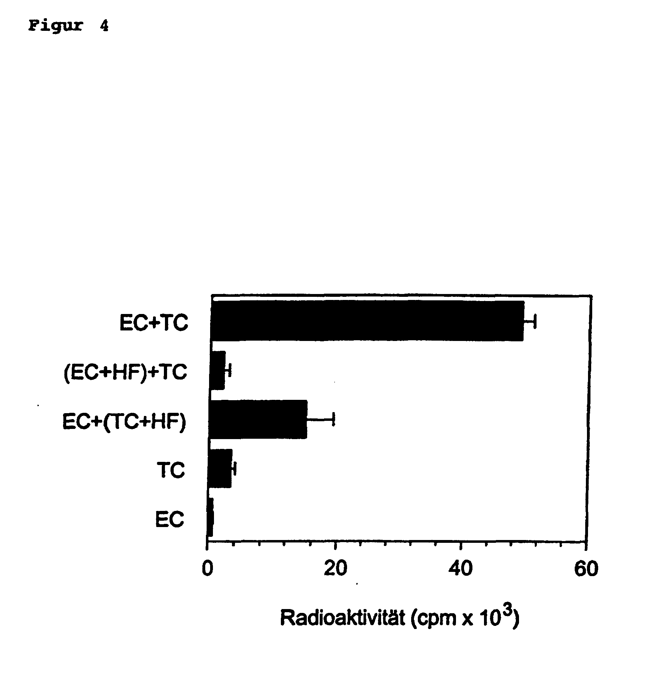

Die Ergebnisse (Figur 4) zeigen, daß Hyperforin sowohl auf EC als auch auf TC eine signifikante Hemmung der Proliferation bewirkt.

**Patentansprüche**

1. Topische Salbe oder Creme, enthaltend mindestens 15 µg/ml Hyperforin.

2. Salbe oder Creme nach Anspruch 1, enthaltend 0,02-20 mg/ml, bevorzugt 1-20 mg/ml Hyperforin.

3. Salbe oder Creme nach einem der Ansprüche 1 oder 2, enthaltend zusätzlich mindestens 15 µg/ml Hypericine.

4. Salbe oder Creme nach Anspruch 3, enthaltend mindestens 5 Gew.-% Johanniskrautextrakt, der mindestens 200 µg/ml Hyperforin und mindestens 200 µg/ml Hypericine enthält.

5. Salbe oder Creme nach Anspruch 4, worin der Johanniskrautextrakt 200-100.000 µg/ml, bevorzugt etwa 1000 µg/ml Hyperforin und 200-2000 µg/ml Hypericine enthält.

**6.** Salbe oder Creme nach Anspruch 4 oder 5, worin der Johanniskrautextrakt 20-60 % v/v Ethanol enthält.

**7.** Salbe nach einem der Ansprüche 3-6, enthaltend etwa 15 Gew.-% Johanniskrautextrakt sowie eine übliche Salbengrundlage.

**8.** Creme nach einem der Ansprüche 3-6, enthaltend etwa 10 Gew.-% Johanniskrautextrakt sowie eine übliche Cremegrundlage.

**9.** Verfahren zur Herstellung einer topischen Salbe oder Creme nach einem der Ansprüche 1-8, worin man Hyperforin und gegebenenfalls Hypericine oder einen Johanniskrautextrakt, der mindestens 200 µg/ml Hyperforin und mindestens 200 µg/ml Hypericine enthält, so mit üblichen pharmazeutisch verträglichen Hilfsstoffen vermischt, dass man eine Salbe oder Creme mit einem Mindestgehalt von 15 µg/ml, bevorzugt 1-20 mg/ml Hyperforin und gegebenenfalls 15 µg/ml Hypericine erhält.

**10.** Verwendung einer Salbe oder Creme nach einem der Ansprüche 1-8 zur Herstellung eines Medikaments zur Behandlung von entzündlichen Hautkrankheiten, Altershaut oder bakteriellen Hautinfektionen.

**11.** Verwendung nach Anspruch 10, worin eine Salbe nach einem der Ansprüche 1-7 zur Behandlung von chronischen, auch superinfizierten Ekzemen, Exsikkationsekzemen, hyperkeratotischen Hand- und Fußekzemen, einer subakuten bis chronischen atopischen Dermatitis (Neurodermitis), Lichen simplex, Kontaktekzemen, Prurigo simplex subacuta und anderen Prurigo-Arten und Psoriasis vulgaris vom Plaque-Typ sowie Altershaut verwendet wird.

**12.** Verwendung nach Anspruch 10, worin eine Creme nach einem der Ansprüche 1-6 oder 8 zur Behandlung einer akuten bis subakuten atopischen Dermatitis (Neurodermitis), eines akuten bis subakuten Kontaktekzems, Psoriasis guttata, der Altershaut oder zur Nachbehandlung und Rezidivprophylaxe aller Ekzeme verwendet wird.

**13.** Verwendung nach Anspruch 10, zur Behandlung von entzündlichen superinfizierten Hauterkrankungen in der Veterinärmedizin.

## Claims

**1.** Topical ointment or cream which comprises at least 15 µg of hyperforin/ml.

**2.** Ointment or cream according to Claim 1 which comprises 0.02-20 mg of hyperforin/ml, preferably 1-20 mg of hyperforin/ml.

**3.** Ointment or cream according to any one of Claims 1 and 2 which additionally comprises at least 15 µg of hypericins/ml.

**4.** Ointment or cream according to Claim 3 which comprises at least 5% by weight of St. John's wort extract which contains at least 200 µg of hyperforin/ml and at least 200 µg of hypericins/ml.

**5.** Ointment or cream according to Claim 4 in which the St. John's wort extract contains 200-100,000 µg/ml, preferably about 1000 µg of hyperforin/ml and 200-2000 µg of hypericins/ml.

**6.** Ointment or cream according to Claim 4 or 5 in which the St. John's wort extract contains 20-60% v/v of ethanol.

**7.** Ointment according to any one of Claims 3-6 which comprises about 15% by weight of St. John's wort extract and also a customary ointment base.

**8.** Cream according to any one of Claims 3-6 which comprises about 10% by weight of St. John's wort extract and also a customary cream base.

**9.** Process for producing a topical ointment or cream according to any one of Claims 1-8, in which process hyperforin and, where appropriate, hypericins, or a St. John's wort extract which contains at least 200 µg of hyperforin/ml

and at least 200 µg of hypericins/ml, is/are mixed with customary pharmaceutically tolerated adjuvants such that an ointment or cream having a minimum content of 15 µg of hyperforin/ml, preferably 1-20 mg of hyperforin/ml, and, where appropriate, 15 µg of hypericins/ml, is obtained.

**10.** Use of an ointment or cream according to any one of Claims 1-8 for the production of a medicament for treating inflammatory skin diseases, geriatric skin or bacterial skin infections.

**11.** Use according to Claim 10 in which an ointment according to one of Claims 1-7 is used for treating chronic, and also superinfected, eczemas, exsiccation eczemas, hyperkeratotic hand and foot eczemas, a subacute to chronic atopic dermatitis (neurodermatitis), lichen simplex, contact eczemas, prurigo simplex subacuta and other prurigo types and psoriasis vulgaris of the plaque type and also geriatric skin.

**12.** Use according to Claim 10 in which a cream according to one of Claims 1-6 or 8 is used for treating an acute to subacute atopic dermatitis (neurodermatitis), an acute to subacute contact eczema, psoriasis guttata or geriatric skin, or for the after-treatment and relapse prophylaxis of all eczemas.

**13.** Use according to Claim 10 for treating inflammatory superinfected skin diseases in veterinary medicine.

## Revendications

**1.** Pommade ou crème topique, contenant au moins 15 µg/ml d'hyperforine.

**2.** Pommade ou crème suivant la revendication 1, contenant 0,02-20 mg/ml, de préférence 1-20 mg/ml, d'hyperforine.

**3.** Pommade ou crème suivant l'une des revendications 1 et 2, contenant en supplément au moins 15 µg/ml d'hypéricines.

**4.** Pommade ou crème suivant la revendication 3, contenant au moins 5% en poids d'extrait de millepertuis, qui contient au moins 200 µg/ml d'hyperforine et au moins 200 µg/ml d'hypéricine.

**5.** Pommade ou crème suivant la revendication 4, dans laquelle l'extrait de millepertuis contient 200-100.000 µg/ml, de préférence environ 1000 µg/ml, d'hyperforine et 200-2000 µg/ml d'hypéricines.

**6.** Pommade ou crème suivant l'une des revendications 4 et 5, dans laquelle l'extrait de millepertuis contient 20-60% v/v d'éthanol.

**7.** Pommade ou crème suivant l'une des revendications 3 à 6, contenant environ 15% en poids d'extrait de millepertuis ainsi qu'une base de pommade courante.

**8.** Crème suivant l'une des revendications 3 à 6, contenant environ 10% en poids d'extrait de millepertuis ainsi qu'une base de crème courante.

**9.** Procédé de préparation d'une pommade ou crème topique suivant une des revendications 1 à 8, dans lequel à de l'hyperforine et éventuellement des hypéricines ou un extrait de millepertuis, qui contient au moins 200 µg/ml d'hyperforine et au moins 200 µg/ml d'hypéricine, on mélange des substances auxiliaires pharmaceutquement compatibles courantes de façon à obtenir une pommade ou crème ayant une teneur minimale de 15 µg/ml, de préférence de 1-20 mg/ml, d'hyperforine et éventuellement 15 µg/ml d'hypéricines.

**10.** Utilisation d'une pommade ou crème suivant la revendication 1 à 8 pour la fabrication d'un médicament destiné au traitement de maladies de la peau inflammatoires, du vieillissement de la peau ou d'infections bactériennes de la peau.

**11.** Utilisation suivant la revendication 10, dans laquelle une pommade suivant l'une des revendications 1 à 7 est utilisée pour le traitement d'eczémas chroniques, ainsi que superinfectés, d'eczémas de dessiccation, d'eczémas de la main et du pied hyperkératotiques, d'une dermatite atopique sub-aiguë à chronique (névrodermite), du Lichen simplex, d'eczémas de contact, de Prurigo simplex subacuta et d'autres types de Prurigo et de Psoriasis vulgaris de type plaque, ainsi que du vieillissement de la peau.

**12.** Utilisation suivant la revendication 10, dans laquelle on utilise une crème suivant l'une des revendications 1 à 6 ou 8, pour le traitement d'une dermatite atopique aiguë à sub-aiguë (névrodermite), d'un eczéma de contact aigu à sub-aiguë, de Psoriasis guttata, du vieillissement de la peau ou pour le traitement ultérieur et la prophylaxie de récidive de tous les eczémas.

**13.** Utilisation suivant la revendication 10, pour le traitement de maladies de la peau superinfectées, inflammatoires, dans la médecine vétérinaire.

Figur 1

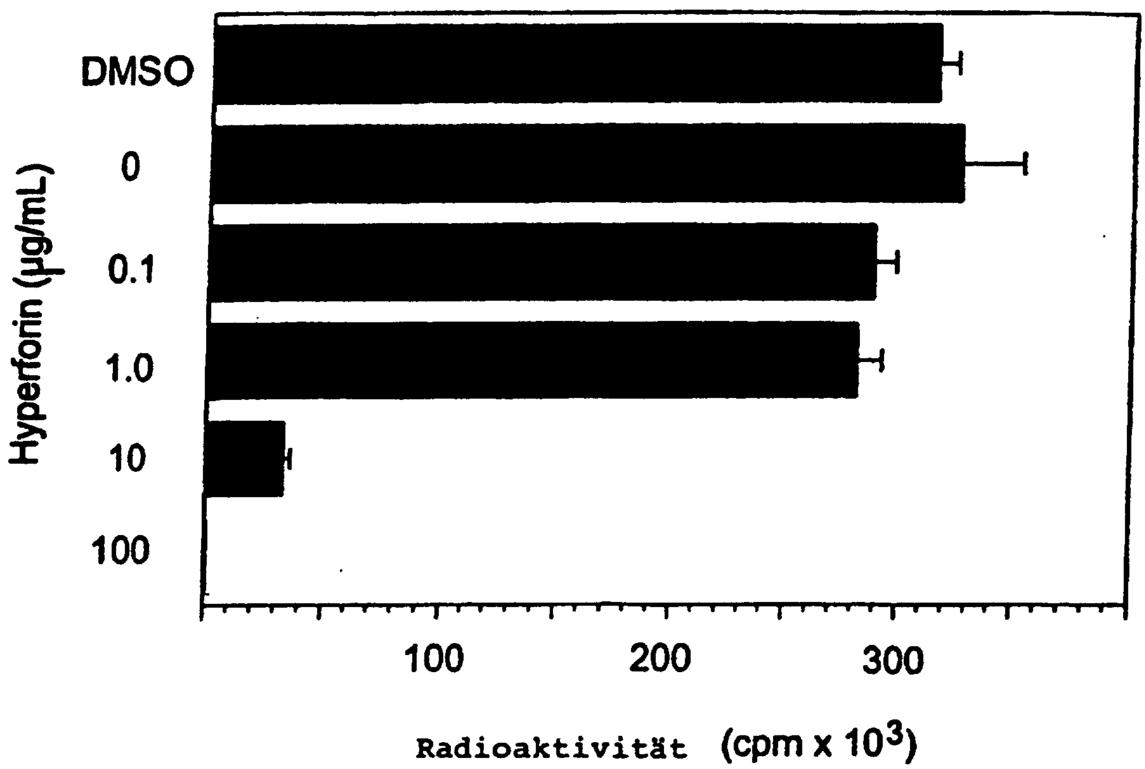
DMSO
0
0.1
1.0
10
100
Hyperforin (µg/mL)
100
200
300
Radioaktivität (cpm x 10³)

Figur 2

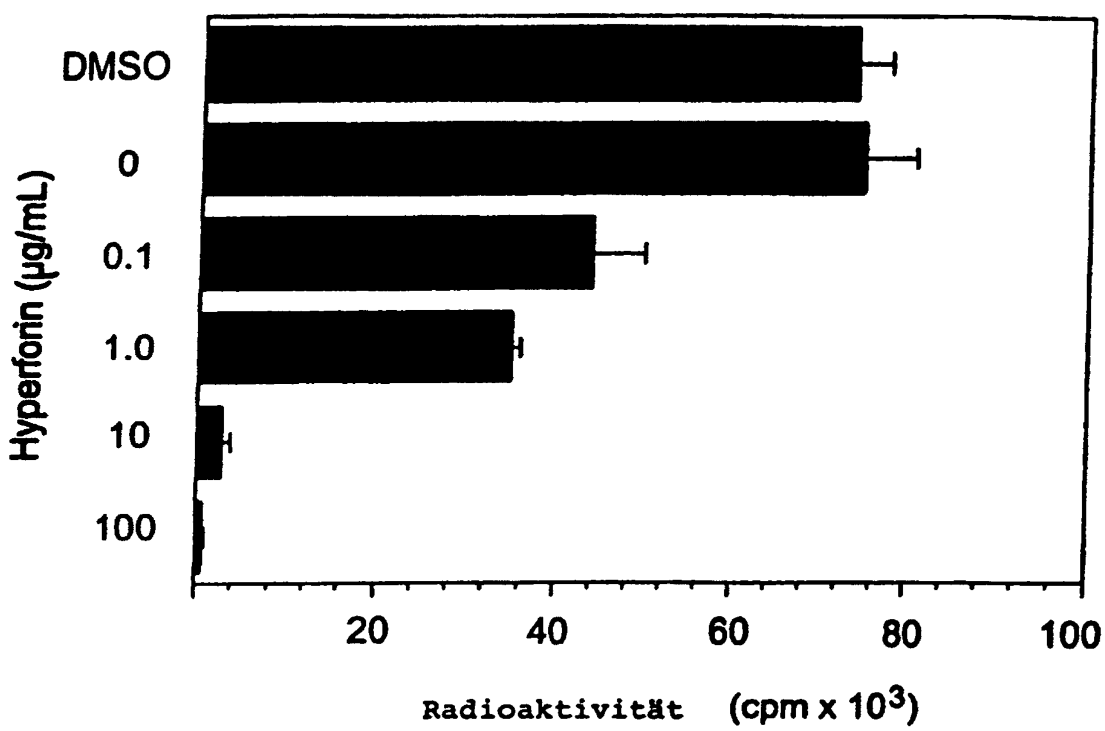
DMSO
0
0.1
1.0
10
100
Hyperforin (µg/mL)
20
40
60
80
100
Radioaktivität (cpm x 10³)

Figur 3

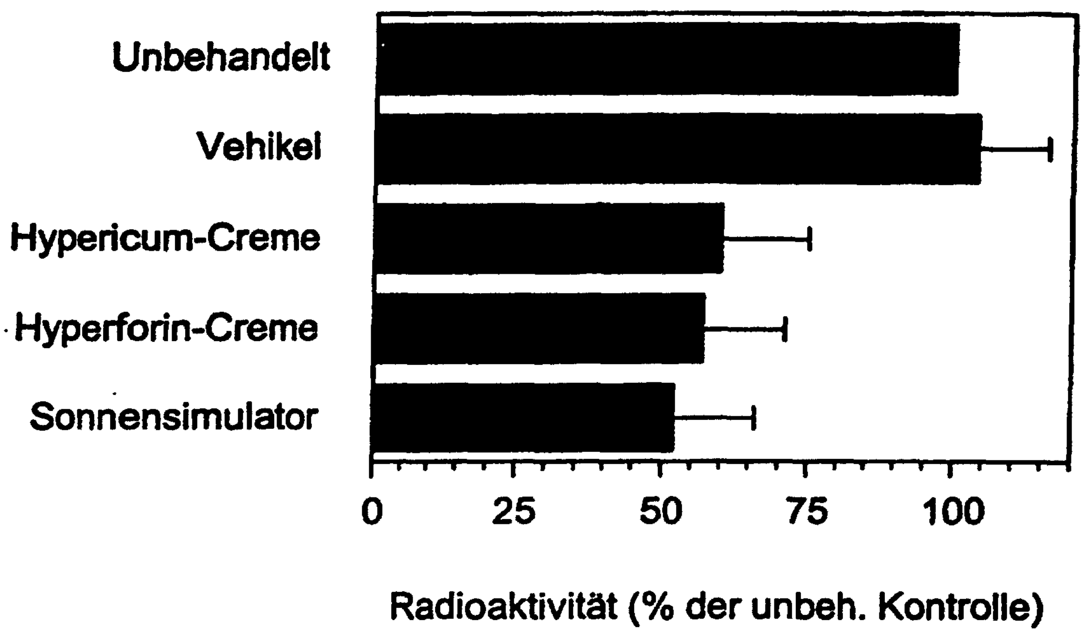
Unbehandelt
Vehikel
Hypericum-Creme
Hyperforin-Creme
Sonnensimulator
0
25
50
75
100
Radioaktivität (% der unbeh. Kontrolle)

Figur 4